# EUROPEAN PATENT SPECIFICATION

(11) **EP 3 937 859 B1**
(45) Date of publication and mention of the grant of the patent: **17.07.2024**
(21) Application number: 20708473.2
(22) Date of filing: 02.03.2020
(51) Int. Cl.: A61F 2/915

(54) **IMPLANT**
IMPLANTAT
IMPLANT

(30) Priority: 15.03.2019 EP 19163172
(43) Date of publication of application: 19.01.2022
(60) Divisional application: 24179517.8
(73) Proprietor: BIOTRONIK AG, 8180 Bülach (CH)
(72) Inventor: SCHOOF, André, 18209 Bad Doberan (DE); FRICKE, Dirk, 18055 Rostock (DE)
(74) Representative: Biotronik Corporate Services SE
(86) International application number: PCT/EP2020/055418
(87) International publication number: WO 2020/187550

(56) References cited:
- EP-A1- 3 388 032
- EP-A2- 1 970 032
- WO-A2-2005/122959
- US-A1- 2017 172 768

## Description

The present invention relates to an implant having an openwork, hollow-cylindrical main body, which is assembled from a plurality of openwork, hollow-cylindrical segments, which are arranged in succession in the longitudinal direction and which are connected to one another, wherein each segment has a plurality of bars, which together form a meandering structure with minimum and maximum points which runs around in the circumferential direction. The implant or main body thereof may assume a compressed state and an expanded state.

A wide variety of medical implants (prostheses), in particular intraluminal endoprostheses, for a wide range of applications are known from the prior art.

Stents are often used as implants and form example may be used for the treatment of stenoses (vascular constrictions). They usually have an openwork, hollow-cylindrical (tubular) main body, which is open at both ends in the longitudinal direction (i.e. in the direction of the longitudinal axis). An implant of this kind is often inserted by means of a catheter into the vessel to be treated and is used to support the vessel by means of its main body over a relatively long period of time (months to years). Narrowed regions in the vessels may be widened by the use of stents.

Implants, in particular stents, which are made partially or wholly of a biodegradable material are also already known. Biodegradation is understood to mean hydrolytic, enzymatic and other metabolically induced breakdown processes in a living organism, which are brought about above all by the bodily fluids coming into contact with the biodegradable material of the implant and lead to a gradual disintegration of the structures of the implant containing the biodegradable material. The implant loses its mechanical integrity at a certain point as a result of this process. The term "biocorrosion" is often used synonymously with the term "biodegradation". The term "bioresorption" includes the subsequent resorption of the degradation products by the living organism.

Document EP 1 974 700 A1 describes an implant in the form of a stent having a radially expandable main body, wherein the main body has a plurality of supporting segments. Each segment is formed by meandering struts. Document EP 3 034 035 A1 also describes such an implant which is flexible and at the same time has a sufficient radial rigidity to support the vessel into which it has been inserted. EP3388032 A1 discloses a stent whereby the radial force of the center is greater than the radial force of the ends of the stent.

Known implants, such as those mentioned above, the main body of which is assembled from a plurality of segments, which are arranged in succession in the longitudinal direction, often have a plurality of struts in each segment, which struts each form a circumferential, meandering structure. The struts for this purpose are fastened to one another or transition into one another at the arcuate maximum and minimum points of the structure. The meandering structure has minimum and maximum points in alternation, wherein the arcs which lie closer to a first end of the main body are referred as the minimum points of a segment, whereas the other arcs, which lie closer to the end of the main body opposite the first end are referred to as the maximum points.

Within the scope of this application, a segment is understood to mean a hollow-cylindrical portion of the implant. The segment consists of a plurality of struts and arcs, which form a circumferential, meandering structure. The segment is closed in the circumferential direction, that is to say the struts and arcs of a segment are connected to one another in such a way that they form a closed ring in the circumferential direction. In the case of a stent, an embodiment of this kind is often also referred to as a ring design.

The known implants on the one hand assume the compressed state already mentioned above, in which they may be introduced minimally-invasively into the body of the patient to be treated, for example by means of a catheter. On the other hand, the implants are transferred into the expanded state, for example by means of balloon dilation, at the treatment site, where the implant shall remain. In the expanded state the implants support the vessel in question by means of their main body or are anchored in the relevant organ or other bodily cavity. Within the scope of the application, the expanded state is understood to mean the state of the implant that is provided following expansion, for example by means of balloon dilation, wherein individual and local external influences brought about by the individual implantation site (for example locally different vessel diameter, rigidities or deviations from a circular cross-section) are not taken into consideration. The expanded state therefore corresponds to a state following expansion in the open air.

Within the scope of the following explanation, the term "vessel" shall include all vessels, organs or other bodily cavities, in particular blood vessels, of a patient to be treated into which a generic implant may be inserted for the treatment.

If an implant having the above-described main body is inserted into a vessel, the bodily liquid of this vessel thus flows through the implant. At the transition between the vessel without implant and vessel with implant, flow changes occur, which prevent the flow of the bodily fluid and may potentially lead to an undesirable depositing or agglomeration of contents of this bodily fluid in the region of this transition. In the case of a vessel that moves (for example a blood vessel), the rigidity of the particular vessel also changes in the region of the inserted implant, since the implant present at the treatment site also contributes to the rigidity. This change to rigidity, where possible, should not be sudden.

The object thus lies in creating an implant which attains an improved flow transition and a smaller change in rigidity at the transition from the vessel without implant to the vessel with implant. Furthermore, the implant according to the invention it should be better and more easily implantable than comparable prior art implants.

The above object is achieved by an implant having the features of claim 1.

In particular, the implant according to the invention has a plurality of openwork, hollow-cylindrical segments arranged in succession in the longitudinal direction, with a meandering structure as described above, wherein the segments each comprise a terminal segment at each end of the main body arranged in the longitudinal direction and at least one inner segment, which is arranged in the longitudinal direction between the two terminal segments. In accordance with the invention the meandering structure of at least one terminal segment has a smaller number of minimum points than the at least one inner segment.

A segment preferably has just as many minimum points as maximum points. This is preferably true equally for both the terminal segments and for the inner segments.

The outer diameter of this at least one terminal segment in the expanded state is preferably substantially equal to the outer diameter of the at least one inner segment in the same state. In other words, a hollow-cylindrical main body of which the outer diameter does not differ between inner segments and end segments is produced when the implant is expanded. Within the scope of this application, "substantially equal outer diameter" is understood to mean a difference in diameter of less than 3%, preferably less than 2%.

Due to the solution according to the invention, an implant is created of which the main body enables an improved implantation procedure. Since the structure of the segments is meandering, the reduction in the number of minimum points in the terminal segment simultaneously also signifies a corresponding reduction in the number of the maximum points and of the bars. This means that the terminal segment may be compressed to a smaller diameter than the inner segments. At the time of implantation, the implant is inserted head-first into the vessel. Due to the smaller compressed diameter of the terminal segment, the risk of the terminal segment sticking out and hitting against and injuring the vessel wall as it is inserted is minimised. In addition, due to the smaller number of bars in the region of the terminal segment, an improved flow transition is created.

In one exemplary embodiment of the invention both terminal segments have a smaller number of minimum points than the at least one inner segment.

In a further exemplary embodiment the number of minimum points of one terminal segment or of both terminal segments is 1 or 2 times smaller than the number of the minimum points of the inner segments. If the inner segments have different numbers of minimum points, then in one specific embodiment the number of minimum points of one terminal segments or of both terminal segments should be 1 or 2 times smaller than the number of minimum points of the inner segment having the smallest number of minimum points.

For example, the implant has inner segments which each form 6 minimum points in the meandering structure. In this exemplary embodiment a terminal segment or both terminal segments for example has/have 4 or 5 minimum points. Other numbers of minimum points are also included by the above definition, wherein the number of minimum points is a natural number.

According to the invention, at least one eyelet for the arrangement of a functional element consisting of an x-ray opaque and/or radiopaque material is provided on the meandering structure of at least one terminal segment, wherein the inner opening of the eyelet preferably has an elliptical form. An eyelet with a round form of the inner opening or a square form or a rectangular form or another form of the inner opening is also conceivable. The arrangement of a comparatively large x-ray opaque and/or radiopaque functional element as compared to conventional implants is possible in the case of the implant according to the invention, since the space necessary for this was created by the reduced number of minimum points and thus reduced number of bars, without hindering the compression or expansion of the main body in this region. The outer length of the eyelet, i.e. the outer dimension in the longitudinal direction, is smaller than or equal here to the width of the terminal segment. Here, the width of a segment (segment width) is considered to be the outer length of the segment in the particular state in the longitudinal direction of the main body. In this embodiment the advantages of the invention in relation to a small diameter in the compressed state are particularly emphasised. Due to the reduced number of minimum points, more space is created for the functional element, such that the implant may be compressed together with functional element as a whole to a smaller diameter. The functional element does not increase the diameter in the compressed state. This leads to a small profile of a catheter system for insertion of an implant of this kind and thus enables a simpler and less traumatic implantation.

The functional element may be made for example of one or more x-ray opaque and/or radiopaque elements or compounds from the group comprising platinum, iridium, gold, tungsten, molybdenum, niobium, tantalum, yttrium, zirconium, ytterbium or alloys of these metals.

In a specific exemplary embodiment a functional element consisting of an x-ray opaque and/or radiopaque material is arranged in the at least one eyelet. The functional element in this case is fastened to the eyelet for example by means an adhesive bond.

In a specific exemplary embodiment a further reduction of the variation in rigidity between vessel with implant and vessel without implant may be achieved in that at least one terminal segment has a segment width that is larger than the segment width of the at least one inner segment. Due to the larger segment width of the at least one terminal segment, this has a further reduced radial rigidity. The segment width of the terminal segment may be for example between 1 mm and 2 mm, preferably between 1.1 mm and 1.6 mm. The segment width of the inner segment may lie for example in a similar range. The inner segment(s) advantageously has/have a smaller bar width between 0.01 mm and 0.3 mm, preferably between 0.01 mm and 0.2 mm, particularly preferably between 0.07 mm and 0.12 mm.

In a further exemplary embodiment the distance between a terminal segment and the adjacent inner segment is greater than the distance between two adjacent inner segments. The axial rigidity in the region of the relevant terminal segment is hereby reduced, and therefore an improved rigidity transition is created. Here, the distance between two segments is understood to be the minimum length of the empty space in the longitudinal direction between the line that connects the minimum points of one segment and the line that connects the opposite maximum points of the other segment. The distance between the terminal segment and the adjacent inner segment in the longitudinal direction may be, for example, between 0.10 mm and 0.13 mm, preferably between 0.11 mm and 0.12 mm. The distance between two adjacent inner segments in the longitudinal direction may be, for example, between 0.08 mm and 0.12 mm, preferably between 0.09 mm and 0.11 mm.

In a further exemplary embodiment the bars of at least one terminal segment have a smaller width and/or a stronger rounding of the edges than the bars of the at least one inner segment. Due to this design of the bars of the segments, an even better flow transition in the direction of the interior of the implant is created, since the material thickness in the region of the terminal region in question is reduced. On the other hand, the radial rigidity in the region of the terminal segment in question is further reduced. The bar width is understood to be the width of the bar measured in a plane that is created when the main body extending over a circumference of a hollow cylinder is unrolled in a plane (unrolling plane). Here, the width of the bar in question is measured in this unrolling plane perpendicularly to the outer faces of the bar lying perpendicularly to the unrolling plane. The smaller width of the bars and/or stronger rounding of the edges in the terminal segment may be achieved for example by an appropriate electropolishing in this region. The bar width of the terminal segment may be, for example, between 0.135 mm and 0.155 mm, preferably between 0.140 mm and 0.150 mm. The bar width of the bars of the inner segment may be, for example, between 0.145 mm and 0.160 mm, preferably 0.150 mm and 0.155 mm. A stronger rounding of the edges is understood within the scope of this application to mean a rounded edge with a larger radius of the arc of the rounding.

In a further exemplary embodiment of the invention the bars of at least one terminal segment have a smaller thickness than the bars of the at least one inner segment. Similarly to the reduction of the bar width, a reduction of the bar thickness results in an improved flow transition in the direction of the implant interior and a reduction of the radial rigidity, whereby an improved transition between the vessel without implant and the vessel with implant is achieved. The bar thickness within the scope of this application will be understood to mean the wall thickness of the bar that results under consideration of the cross-section of a bar perpendicularly to the bar width. A smaller bar width in the terminal element in question may be achieved likewise by a suitable electropolishing. The bar thickness of the terminal segment may be, for example, 5 µm to 15 µm smaller than the bar thickness of an inner segment.

The two embodiments described above are preferably combined. In this preferred combination the bars of at least one terminal segment have a smaller width, a smaller thickness and/or a stronger rounding of the edges.

In a further exemplary embodiment, adjacent segments are connected to one another by means of a connecting bar, wherein the at least one connecting bar has a smaller bar width and/or bar thickness than the bars of the at least one inner segment and/or the bars of the at least one terminal segment. The implant hereby advantageously achieves, on the whole, good axial flexibility and good bending flexibility. The bar width of the connecting bars may be, for example, between 0.07 mm and 0.10 mm, preferably between 0.08 mm and 0.09 mm and is measured analogously to the width of the bars of the segments. Reference can be made to the description above for exemplary bar widths of the bars of the at least one terminal segment and of the bars of the at least one inner segment.

The main body of the implant according to the invention may consist wholly or partially of a biodegradable material. Biodegradable materials of this kind are metal biodegradable materials, in particular based on magnesium or a magnesium alloy, for example WE43, magnesium-zinc-aluminium, magnesium-aluminium or magnesium-zinc-calcium. Here, preferably highly pure magnesium alloys are used, such as magnesium-zinc-aluminium with 0 - 4% by weight Zn and 2 - 10% by weight Al or with 1.5 - 7% by weight Zn and 0.5 - 3.5% by weight Al, such as magnesium-aluminium with 5 - 10% by weight Al, in particular 5.5-7% by weight, particularly preferably 6.25% aluminium, such as magnesium-zinc-calcium with 3 - 7% by weight Zn and 0.001 - 0.5% by weight Ca or 0 - 3% by weight Zn and 0 - 0.6% by weight Ca. Highly pure magnesium alloys of this kind, in addition to the aforesaid alloy elements, also comprise less than 0.006% by weight of other elements (impurities such as Fe, Cu, Co, Si etc. or rare earths). The use of highly pure magnesium alloys of this kind for the main body of the implant has the advantage that the formation of secretions may be very well controlled. By controlling the size, the volume and the composition of the secretions, the degradation behaviour of the implant may be very precisely controlled in turn. The degradation behaviour of the implant is set in such a way that its supporting function is maintained for a period of at least 90 days after implantation into the vessel, and the implant degrades almost fully, that is to say is broken down, after a period of approximately one year.

Examples of suitable biodegradable polymeric compounds are polymers from the group: cellulose, collagen, albumin, casein, polysaccharide (PSAC), polylactide (PLA), poly-L-lactide (PLLA), polyglycol (PGA), poly-D,L-lactide-co-glycolide (PDLLA-PGA), polyhydroxybutyric acid (PHB), polyhydroxyvaleric acid (PHV), polyalkylcarbonate, polyorthoester, polyethylene terephthalate (PET), polymalonic acid (PML), polyanhydride, polyphosphazene, polyamino acids and copolymers thereof and hyaluronic acid. The polymers may be present in pure form, in derivatised form, in the form of blends or as copolymers, depending on the desired properties. The biodegradable material may also consist partially of a metal biodegradable material and partially of a polymeric biodegradable compound.

In an alternative embodiments the main body of the implant is formed wholly or partially from a non-biodegradable metal or metal alloy, such as stainless steel, titanium, cobalt-chromium alloy, nickel-titanium alloy, platinum or similar suitable metal alloys.

In a further preferred embodiment the implant comprises a active ingredient-containing polymer coating at least in part, wherein in particular the entire main body is coated uniformly, i.e. there is no coating difference between the end segments and the inner segments. The polymers mentioned above, in particular polylactide (PLA) or poly-L-lactide (PLLA), may be used as polymers.

Antiproliferative, antimigratory, antiangiogenic, anti-inflammatory, antiphlogistic, cytostatic, cytotoxic and/or antithrombotic active ingredients, anti-restenosis active ingredients, corticoids, sexual hormones, statins, epothilones, prostacyclins, angiogenesis inducers may be used as active ingredient. Paclitaxel and its derivatives or sirolimus and its derivatives are particularly preferred.

The drawings show, schematically:
- Fig. 1: a main body of an implant according to the invention in the state after electropolishing, shown in an unrolling plane in a view from above,
- Fig. 2: separately, the detail D from Fig. 1 in an enlarged view,
- Fig. 3: separately, the detail C from Fig. 1 in an enlarged view,
- Fig. 4: separately, the detail B from Fig. 1 in an enlarged view, and
- Fig. 5: shows an implant according to the invention in the state after electropolishing in a perspective view from the side.

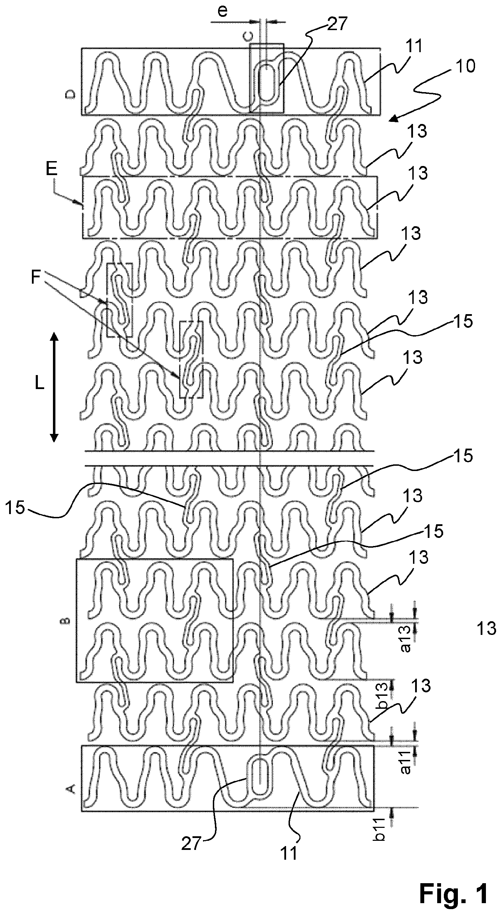

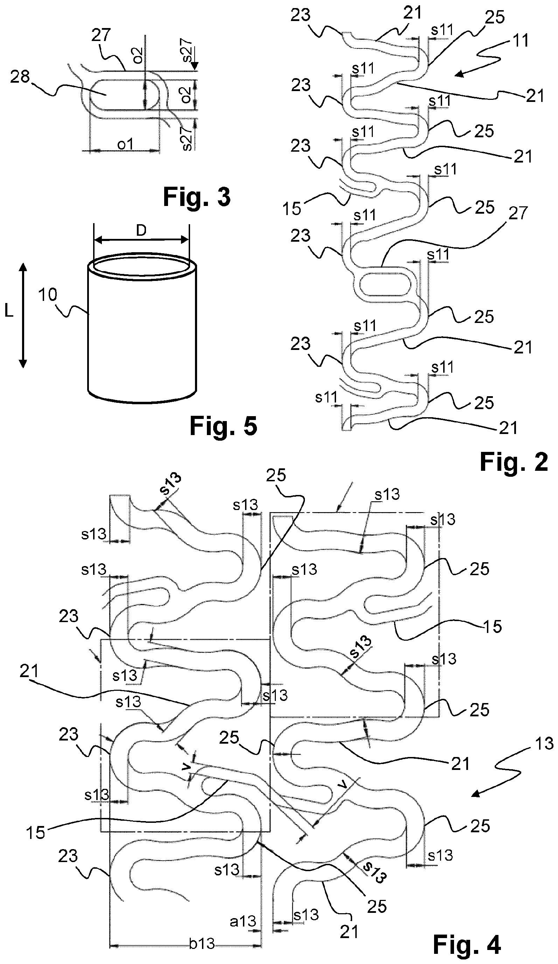

Fig. 1 shows a main body 10 of an exemplary embodiment of an implant according to the invention in the form of a stent in the state after electropolishing, before compression, wherein, for improved illustration of the main body 10, which has an openwork structure with a plurality of bars arranged in individual segments, it has been unrolled in a rolling plane. Fig. 5 shows the hollow-cylinder form of the main body 10 in the compressed state when this has not been unrolled, without showing the structure of the main body 10 in detail.

In the state after electropolishing, the main body 10 has a diameter which is close to the expanded diameter, but is smaller than the target diameter in the expanded state. The diameter of the main body corresponds here substantially to the diameter of the tube from which the main body was produced.

The main body 10 is assembled from a plurality of segments 11, 13, wherein in Fig. 1 a total of eleven segments 11, 13 are shown in full. The segments with the reference sign 11 form terminal segments, since they are provided at the ends of the main body 10 arranged in the longitudinal direction L (direction of the longitudinal axis of the hollow-cylindrical main body 10; see Fig. 5). The other segments 13 form inner segments.

Each segment 11, 13 is connected to its adjacent segment 11, 13 by two connecting bars 15. The connecting bars 15 are highlighted in the details F of the main body 10 in Fig. 1. The connecting bars 15 run in the shown exemplary embodiment in a curved or S shape, but may also run straight in their middle part. In alternative embodiments just a single connecting bar or more than two connecting bars may also be provided for the connection of adjacent segments 11, 13.

Each segment 11, 13 has a plurality of bars, which extend substantially in the longitudinal direction L or at a slight incline thereto. The bars which are provided in Fig. 2 and 4 with the reference sign 21 may run straight or arched or curved, wherein different straight and arched and curved bars may be arranged in a single segment. Each two adjacent bars 21 are connected by arcs, so that a meandering structure is created. The arcs each form either a minimum point 23 or a maximum point 25 of the meandering structure (see Fig. 2 and 4).

In the shown exemplary embodiment (see in particular details A, D and E of Fig. 1 and Fig. 2), the inner segments 13 each have twelve curved bars 21 and twelve arcs, wherein six of these arcs form maximum points 25 and six arcs form minimum points 23. The terminal segments 11 are assembled, by contrast, from ten bars 21 and ten arcs, wherein five arcs form maximum points 25 and five arcs form minimum points 23. An eyelet 27 is arranged on a bar 21 of the terminal segment 11 and is shown on an enlarged scale in Fig. 3. Here, the stent according to the invention has the same or substantially the same outer diameter both in the region of the inner segments 13 and in the region of the terminal segments 11 in the state after electropolishing, and this outer diameter is denoted by D in Fig. 5. In the expanded state the inner segments 13 have the same or substantially the same inner diameter as the end segments 11. Minor differences in the outer diameter D between the terminal segments 11 and the inner segments 13 may be produced merely as a result of a different springback behaviour after the compression or after the expansion on account of the different mechanical characteristics of the inner segments 13 and the terminal segments 11. The outer diameter D for the inner segments 13 and the terminal segments 11 is substantially the same.

Other numbers of minimum points, maximum points and bars in the inner segments and in the terminal segments are likewise conceivable, wherein, in accordance with the invention, the number of the minimum points of the terminal segments is smaller by at least one than the number of the minimum points of the inner segments.

The eyelet 27 shown in detail in Fig. 3 is used for the arrangement of a functional element consisting of radiopaque and/or x-ray opaque material in the inner, continuous opening 28, so as to ensure the visibility of the stent as it is introduced into the body. Due to the reduction of the minimum points in the terminal segments, it is possible to increase the volume or the visible area of the functional element, such that the visibility of the stent is improved. Here, the eyelet 27 and its inner opening 28 have an approximately elliptical form, which has a large area as compared with other forms. The ellipse is arranged in the main body 10 in such a way that its main axis runs parallel to the longitudinal direction L of the main body. In addition, the main axes of the elliptical openings 28 in the two eyelets 27 arranged on the two terminal segments 11 are offset by a length e in the circumferential direction (see Fig. 1). The length of the main axis o1 of the opening 28 of the eyelet 27 is for example 800 µm, and the length of the auxiliary axis o2 is for example 350 µm. The bar widths s 27 of the eyelet are for example 100 µm.

The length of the main axis o1 of the opening 28 of the eyelet 27 is for example 800 µm, and the length of the auxiliary axis o2 is for example 350 µm. The bar widths s27 of the eyelet are for example 100 µm.

The two terminal segments 11 have a segment width b11 of, for example, 1.36 mm, whereas the segment width b13 of the inner segments 13 is, for example, 1.25 mm (see Fig. 1). Other segment widths are also conceivable, wherein preferably: b11 > b13. The radial rigidity of the terminal segments 11 is hereby further reduced.

The distances between the segments are also shown in Fig. 1. Reference sign a11 denotes the distance between a terminal segment 11 and an adjacent, inner segment 13, whereas a13 denotes the distance between two inner segments 13. In order to reduce the axial rigidity in the transition region, a11 > a13, for example a11 = 115 µm and a13 = 95 µm in the shown exemplary embodiment of an implant.

A better transition in the rigidity from the unstented vessel to the stented vessel is also attained if the bar widths s11 of the terminal segments 11 are smaller than the bar widths s13 of the inner segments, wherein the segments may have different bar widths s11, s13 within a segment. For example, s11 is between 135 µm and 149 µm and s13 is between 150 µm and 165 µm. The same is true for different bar thicknesses (not shown) between the bars of the terminal segments 11 and the bars of the inner segments 13.

Furthermore, in the shown exemplary embodiment of an implant, in order to ensure good axial flexibility and good bending flexibility of the stent or main body 10, the bar width v of the connecting bars 15 is smaller than the bar width s13 of the bars of the inner segments 13 and the bar width s11 of the terminal segments 11. For example, the bar width v of the connecting bars 15 is between 80 µm and 90 µm.

In addition, the bars 21, maximum points 25 and minimum points 23 of the terminal segments 11 have a stronger rounding of the edges than the same elements of the meandering structure of the inner segments 13. The disruption to the flow of the bodily fluid at the transition to the stent is hereby also reduced.

The stent according to the invention may be produced in the known manner by means of laser cutting from a tubular semi-finished product and subsequent electropolishing. A functional element formed similarly to the inner opening 28 of the eyelet and made of x-ray opaque and/or radiopaque material is then inserted into the opening 28 and is fastened to the eyelet 27, for example by means of adhesive bonding.

In order to treat a patient, the stent according to the invention for example may be crimped onto the balloon of a catheter (not shown). The stent may then be introduced by means of the catheter into the body of the patient, and for example advanced along blood vessels to the point that is to be treated. The stent is then expanded by means of the balloon, and is hereby fastened to the wall of the vessel. The stent is used to hold the vessel open and to support it. The catheter is removed following the anchoring of the stent in the vessel.

The main body of the stent consists of a highly pure magnesium-aluminium alloy containing 5.5 - 7% by weight aluminium (preferably 6.25% by weight aluminium), at most 0.006% by weight of other elements (impurities such as Fe, Cu, Co, Si etc. or rare earths) and magnesium as remaining constituent. The stent also has a sirolimus-containing PLLA coating, wherein the coating on the luminal side (inner side) of the stent if thinner (2-7 µm) than on the abluminal side (outer side) of the stent (10 - 15 µm).

## Claims

1. An implant having an openwork, hollow-cylindrical main body (10), which is assembled from a plurality of openwork, hollow-cylindrical segments (11, 13), which are arranged in succession in the longitudinal direction (L) and which are connected to one another, wherein the segments comprise a terminal segment (11) at each end of the main body (10) arranged in the longitudinal direction and at least one inner segment (13), which is arranged in the longitudinal direction (L) between the two terminal segments (11), wherein each segment (11, 13) has a plurality of bars (21), which together form a meandering structure with maximum points (25) and minimum points (23) which runs around in the circumferential direction, wherein the main body (10) assumes a compressed state and an expanded state, **wherein** the meandering structure of at least one terminal segment (11) has a smaller number of minimum points (23) than the at least one inner segment (13), and **characterised by** at least one eyelet (27) for the arrangement of a functional element consisting of an x-ray opaque and/or radiopaque material is provided in the meandering structure of at least one terminal segment (11) of the main body (10).

2. The implant according to claim 1, **characterised in that** the outer diameter of the at least one terminal segment in the expanded state corresponds to the outer diameter of the at least one inner segment.

3. The implant according to either one of the preceding claims, **characterised in that** both terminal segments (11) have a smaller number of minimum points (23) than the at least one inner segment (13).

4. The implant according to any one of the preceding claims, **characterised in that** the inner opening (28) of the eyelet (27) has an elliptical form.

5. The implant according to claim 4, **characterised in that** a functional element consisting of an x-ray opaque and/or radiopaque material is arranged in the at least one eyelet (27).

6. The implant according to any one of the preceding claims, **characterised in that** at least one terminal segment (11) has a segment width (b11) which is greater than the segment width (b13) of the at least one inner segment (13).

7. The implant according to any one of the preceding claims, **characterised in that** the distance (a11) between a terminal segment (11) and the adjacent inner segment (13) is greater than the distance (a13) between two adjacent inner segments (13).

8. The implant according to any one of the preceding claims, **characterised in that** the bars (21) of at least one terminal segment (11) have a smaller width (s11) and/or thickness and/or a stronger rounding of the edges than the bars (21) of the at least one inner segment (13).

9. The implant according to any one of the preceding claims, **characterised in that** adjacent segments (11, 13) are connected to one another by means of at least one connecting bar (15), wherein the at least one connecting bar (15) has a smaller bar width (v) than the bars (21) of the at least one inner segment (13) and/or the bars of the at least one terminal segment.

## Patentansprüche

1. Implantat mit einem offenen, hohlzylinderförmigen Hauptkörper (10), der aus einer Vielzahl offener hohlzylinderförmiger Segmente (11, 13) zusammengesetzt ist, die aufeinanderfolgend in Längsrichtung (L) angeordnet sind und die miteinander verbunden sind, wobei die Segmente ein Abschlusssegment (11) an jedem in Längsrichtung angeordneten Ende des Hauptkörpers (10) und mindestens ein inneres Segment (13), das in Längsrichtung (L) zwischen den zwei Abschlusssegmenten (11) angeordnet ist, umfassen, wobei jedes Segment (11, 13) eine Vielzahl Stege (21) aufweist, die zusammen eine mäandernde Struktur mit Hochpunkten (25) und Tiefpunkten (23) bilden, die in umlaufender Richtung verlaufen, wobei der Hauptkörper (10) einen komprimierten Zustand und einen expandierten Zustand annimmt, **wobei** die mäandernde Struktur von mindestens einem Abschlusssegment (11) eine geringere Anzahl von Tiefpunkten (23) aufweist als das mindestens eine innere Segment (13), und **dadurch gekennzeichnet, dass** mindestens eine Öse (27) zur Anordnung eines funktionalen Elements, das aus einem röntgenopaken und/oder strahlenundurchlässigen Material besteht, in der mäandernden Struktur von mindestens einem Abschlusssegment (11) des Hauptkörpers (10) vorhanden ist.

2. Implantat nach Anspruch 1, **dadurch gekennzeichnet, dass** der Außendurchmesser des mindestens einen Abschlusssegments im expandierten Zustand dem Außendurchmesser des mindestens einen inneren Segments entspricht.

3. Implantat nach einem der vorstehenden Ansprüche, **dadurch gekennzeichnet, dass** beide Abschlusssegmente (11) eine geringere Anzahl von Tiefpunkten (23) als das mindestens eine innere Segment (13) aufweisen.

4. Implantat nach einem der vorstehenden Ansprüche, **dadurch gekennzeichnet, dass** die innere Öffnung (28) der Öse (27) eine elliptische Form hat.

5. Implantat nach Anspruch 4, **dadurch gekennzeichnet, dass** ein funktionales Element, das aus einem röntgenopaken und/oder strahlenundurchlässigen Material besteht, in der mindestens einer Öse (27) angeordnet ist.

6. Implantat nach einem der vorstehenden Ansprüche, **dadurch gekennzeichnet, dass** mindestens ein Abschlusssegment (11) eine Segmentbreite (b11) aufweist, die größer als die Segmentbreite (b13) des mindestens einen inneren Segments (13) ist.

7. Implantat nach einem der vorstehenden Ansprüche, **dadurch gekennzeichnet, dass** der Abstand (a11) zwischen einem Abschlusssegment (11) und dem benachbarten inneren Segment (13) größer als der Abstand (a13) zwischen zwei benachbarten inneren Segmenten (13) ist.

8. Implantat nach einem der vorstehenden Ansprüche, **dadurch gekennzeichnet, dass** die Stege (21) von mindestens einem Abschlusssegment (11) eine geringere Breite (s11) und/oder Dicke und/oder eine ausgeprägtere Rundung der Kanten aufweisen als die Stege (21) des mindestens einen inneren Segments (13).

9. Implantat nach einem der vorstehenden Ansprüche, **dadurch gekennzeichnet, dass** benachbarte Segmente (11, 13) miteinander mittels mindestens eines Verbindungsstegs (15) verbunden sind, wobei der mindestens eine Verbindungssteg (15) eine kleinere Stegbreite (v) als die Stege (21) des mindestens einen inneren Segments (13) und/oder die Stege des mindestens einen Abschlusssegments aufweist.

## Revendications

1. Implant ayant un corps principal (10) creux, cylindrique, à cadre ouvert, qui est assemblé à partir d'une pluralité de segments (11, 13) creux, cylindriques, à cadre ouvert, qui sont agencés les uns à la suite des autres dans la direction longitudinale (L) et qui sont reliés les uns aux autres, dans lequel les segments comprennent un segment terminal (11) à chaque extrémité du corps principal (10) agencé dans la direction longitudinale et au moins un segment intérieur (13), qui est agencé dans la direction longitudinale (L) entre les deux segments terminaux (11), dans lequel chaque segment (11, 13) a une pluralité de barres (21), qui forment ensemble une structure sinueuse avec des points maximaux (25) et des points minimaux (23) qui fait le tour dans la direction de la circonférence, dans lequel le corps principal (10) adopte un état comprimé et un état étendu, **dans lequel** la structure sinueuse d'au moins un segment terminal (11) a un nombre de points minimaux (23) inférieur à celui de l'au moins un segment intérieur (13), et **caractérisé par le fait qu'**au moins un oeillet (27) pour l'agencement d'un élément fonctionnel constitué d'un matériau opaque aux rayons X et/ou radio-opaque est pourvu dans la structure sinueuse d'au moins un segment terminal (11) du corps principal (10).

2. Implant selon la revendication 1, **caractérisé en ce que** le diamètre extérieur de l'au moins un segment terminal dans l'état étendu correspond au diamètre extérieur de l'au moins un segment intérieur.

3. Implant selon l'une ou l'autre des revendications précédentes, **caractérisé en ce que** les deux segments terminaux (11) ont un nombre de points minimaux (23) inférieur à celui de l'au moins un segment intérieur (13).

4. Implant selon l'une quelconque des revendications précédentes, **caractérisé en ce que** l'ouverture intérieure (28) de l'oeillet (27) a une forme elliptique.

5. Implant selon la revendication 4, **caractérisé en ce qu'**un élément fonctionnel constitué d'un matériau opaque aux rayons X et/ou radio-opaque est agencé dans l'au moins un oeillet (27).

6. Implant selon l'une quelconque des revendications précédentes, **caractérisé en ce qu'**au moins un segment terminal (11) a une largeur de segment (b11) qui est supérieure à la largeur de segment (b13) de l'au moins un segment intérieur (13).

7. Implant selon l'une quelconque des revendications précédentes, **caractérisé en ce que** la distance (a11) entre un segment terminal (11) et le segment intérieur (13) adjacent est supérieure à la distance (a13) entre deux segments intérieurs (13) adjacents.

8. Implant selon l'une quelconque des revendications précédentes, **caractérisé en ce que** les barres (21) d'au moins un segment terminal (11) ont une largeur (s11) inférieure et/ou une épaisseur inférieure et/ou un arrondi des bords plus prononcé que ceux des barres (21) de l'au moins un segment intérieur (13).

9. Implant selon l'une quelconque des revendications précédentes, **caractérisé en ce que** les segments (11, 13) adjacents sont reliés l'un à l'autre au moyen d'au moins une barre de liaison (15), dans lequel l'au moins une barre de liaison (15) a une largeur de barre (v) inférieure à celle des barres (21) de l'au moins un segment intérieur (13) et/ou des barres de l'au moins un segment terminal.
